# EUROPÄISCHE PATENTSCHRIFT

(11) **EP 1 226 310 B1**
(45) Veröffentlichungstag und Bekanntmachung des Hinweises auf die Patenterteilung: **26.11.2003**
(21) Anmeldenummer: 00979482.7
(22) Anmeldetag: 30.09.2000
(51) Int. Cl.: D21H 27/30, D21H 21/22

(54) **TISSUE- UND/ODER TISSUEÄHNLICHES MATERIAL ZUR HERSTELLUNG VON KÜCHENPAPIER, TOILETTENPAPIER, TASCHENTÜCHER, SAUGEINLAGEN UND ÄHNLICHEM**
TISSUE MATERIAL AND/OR TISSUE-LIKE MATERIAL FOR PRODUCING KITCHEN PAPER, TOILET PAPER, FACIAL TISSUES, ABSORBENT INSERTS AND THE LIKE
PAPIER MENAGER ET/OU MATIERE DE TYPE PAPIER MENAGER UTILISE(E) POUR PRODUIRE DU PAPIER ESSUIE-TOUT, DU PAPIER HYGIENIQUE, DES MOUCHOIRS EN PAPIER, DES GARNITURES HYGIENIQUES ET SIMILAIRES

(30) Priorität: 06.10.1999 DE 19947942
(43) Veröffentlichungstag der Anmeldung: 31.07.2002
(73) Patentinhaber: Metsä Tissue Oyj, 02100 ESPOO (FI)
(72) Erfinder: ZEILER, Christoph, 56316 Raubach (DE)
(74) Vertreter: Geskes, Christoph, Dr.
(86) Internationale Anmeldenummer: EP0009614
(87) Internationale Veröffentlichungsnummer: WO01025537

(56) Entgegenhaltungen:
- WO-A-97/11227
- DE-A- 19 909 835
- GB-A- 1 096 836

## Beschreibung

Die vorliegende Erfindung betrifft ein Tissue- und/oder tissueähnliches Material zur Herstellung von Küchenpapier, Toilettenpapier, Taschentücher, Saugeinlagen und ähnlichem sowie ein Verfahren zur Herstellung desselben und dessen Verwendung.

Im Sinne der Erfindung wird unter Tissue ein Erzeugnis verstanden, welches der DIN 6730 bzw. ISO 4046-1978 6.150 entspricht. Demnach ist Tissue ein Erzeugnis, welches ganz oder überwiegend aus Zellstoffasern besteht, mit einer feinen und weichen Kreppung und geschlossener Formation in der Papiermaschine mit einem Trockengehalt von mehr als 90% gekreppt wird, aus einer oder mehreren Lagen besteht, besonders saugfähig ist, eine flächenbezogene Masse der Einzellage von < 25 g/m² vor der Kreppung und eine Kreppnaßdehnung > 5% aufweist.

Im Sinne der Erfindung wird unter tissueähnlichem Material ein Material verstanden, welches im Unterschied zu Tissue-Material durch Veränderungen im Trockenvorgang hergestellt wird. Im Sinne der Erfindung wird unter tissueähnlichem Material auch Zellstoffwatte gemäß DIN 6730 bzw. ISO 4046-19786.149 verstanden. Demnach ist Zellstoffwatte ein Erzeugnis überwiegend aus Zellstoffasern mit großer und weicher Kreppung und offener Formation, welches in der Papiermaschine trockengekreppt wird und aus einer oder mehreren Lagen besteht, wobei die flächenbezogene Masse einer Lage vor der Kreppung < 25 g/m² und die Naßkreppung einer Lage nach der Kreppung > 25% ist. Zellstoffwatte ist besonders saugfähig.

Tissue- und/oder tissueähnliche Materialien werden für die Herstellung einer Vielzahl von Produkten verwendet. Insbesondere werden derartige Materialien zur Herstellung von Küchenpapieren, Toilettenpapieren, Taschentüchern, Saugeinlagen, insbesondere für Fleisch, Papierhandtüchern und ähnlichem verwendet. Je nach Verwendungszweck werden an Tissue- und/oder tissueähnliche Materialien unterschiedliche Anforderungen gestellt. So steht bei Taschentüchern und Kosmetiktüchern insbesondere deren Weichheit im Vordergrund, wohingegen bei Papierhandtüchern die Festigkeit eine wesentliche Eigenschaft darstellt.

Bei Küchenpapieren oder anderen saugfähigen Papieren wird allgemein ein hohes Absorptionsvermögen im Hinblick auf die aufzunehmende Feuchtigkeit, insbesondere Wasser, und eine gute Naßfestigkeit verlangt. Ein hohes Absorptionsvermögen für Flüssigkeiten und damit eine hohe Saugleistung geht im allgemeinen einher mit einem hohen Volumen der verwendeten Materialien. Dabei kann dieses Volumen unter anderem durch Prägung als auch durch eine Mehrlagigkeit der aus dem Material hergestellten Lagen erzeugt werden.

So ist es bekannt, zwei oder mehrere Lagen aus Tissue-Material miteinander zu verbinden und zu prägen. Hierdurch wird das Volumen und damit auch das Absorptionsvermögen der solchermaßen hergestellten Tissue-Produkte vergrößert. Auf diese Art und Weise werden beispielsweise Küchenpapierrollen hergestellt.

Das Volumen kann jedoch auch durch eine spezielle Behandlung der Einzellagen gesteigert werden. Ein Beispiel hierfür ist ein zweilagiges Küchenpapier, wobei dessen Einzellagen aus Tissue-Material mittels Durchströmtrockung hergestellt werden. Die solchermaßen hergestellten Einzellagen sind tissueähnliche Materialien. Bei der Durchströmtrockung wird im Unterschied zur konventionellen Kontakttrocknung in Tissue-Maschinen die Tissuebahn über einer rotierenden Trommel von Heißluft durchströmt und dadurch getrocknet. Die Tissuebahn wird dabei durch Trockensiebe geführt und gestützt. Durch diese schonende Trocknung, durch Aufbringung eines Prägemusters mittels der verwendeten Siebe sowie verminderter Pressung im Produktionsprozeß entsteht gegenüber normalem Tissue ein tissueähnliches Material mit einem erhöhten spezifischen Volumen. Das übliche spezifische Volumen von normalem Tissue-Material liegt in einem Bereich von etwa 6 bis 7 cm³/g.

Nachteilig an dem vorstehend beschriebenen Verfahren der Durchströmtrocknung ist insbesondere, daß dieses nicht auf konventionellen Tissue-Maschinen, welche in einem jeden papierverarbeitenden Betrieb vorhanden sind, durchgeführt werden kann. Des weiteren verbrauchen Tissue-Maschinen mit Durchströmtrocknung aufgrund der schonenderen Trockungsmethode eine größere Energiemenge als konventionelle Tissue-Maschinen, was sich letzten Endes in erhöhten Kosten bei den Endprodukten niederschlägt.

WO-A-97/11227 offenbart ein mehrlagiges bahnförmiges Tissueprodukt mit wenigstens drei miteinander wenigstens zum Teil verbundenen Lagen, mit zwei gegebenenfalls geprägten, überwiegend Zellstoff enthaltenden Außenlagen und wenigstens einer gegebenenfalls geprägten überwiegend HTCTMP/CTMP enthaltenden Innenlage, wobei sonstige Holzstoffe und/oder Zellstoff-Fasern, die in der Art und Weise chemisch versteift oder innerhalb der Faserwand quervernetzt wurden, daß die Fähigkeit dieser Fasern zur Ausbildung von Wasserstoffbrückenbindungen im feuchten Zustand signifikant reduziert ist, ebenfalls zum Einsatz kommen können, dadurch gekennzeichnet, daß nur die Außenlage 0,1 bis 10 Gew% eines Naßfestmittels, bezogen auf das Rohtissue, enthalten, sowie ein Verfahren zur Herstellung eines mehrlagigen bahnförmigen Tissueprodukts mit wenigstens drei miteinander wenigstens zum Teil verbundenen Lagen, mit zwei gegebenenfalls geprägten überwiegend Zellstoff enthaltenden Außenlagen und wenigstens einer gegebenenfalls geprägten überwiegend HTCTMP/CTMP enthaltenden Innenlage, wobei sonstige Holzstoffe und/oder Zellstoff-Fasern, die in der Art und Weise chemisch versteift oder innerhalb der Faserwand quervernetzt wurden, daß die Fähigkeit dieser Fasern zur Ausbildung von Wasserstoffbrückenbindungen im feuchten Zustand signifikant reduziert ist, ebenf alls zum Einsatz kommen können.

GB-A-1 096 836 offenbart ein absorbierendes Erzeugnis mit hohem Flüssigkeitsaufnahmevermögen, geeignet für Windeln, Binden, Kompressen, Damenbinden und der dergleichen, wobei das Erzeugnis zwischen den Schichten eines Zellstoffprodukts einen kolloidalen Zelluloseether, verteilt in Pulver- oder Kornform, nicht als Fasern, umfaßt, der mit Wasser quillt und dadurch eine große Flüssigkeitsmenge binden kann.

Aufgabe der vorliegenden Erfindung ist es, ein Material zur Verfügung zu stellen, welches ein hohes Absorptionsvermögen aufweist und auf konventionellen Tissue-Maschinen herstellbar ist.

Diese Aufgabe wird gemäß der Erfindung dadurch gelöst, daß ein Material zur Herstellung von Küchenpapier, Toilettenpapier, Taschentüchern, Saugeinlagen und ähnlichem zur Verfügung gestellt wird, welches mindestens dreilagig ist und zwei Außenlagen und mindestens eine zwischen diesen beiden Außenlagen angeordnete Innenlage mit einem höheren Absorptionswert für Wasser als die Außenlagen umfaßt, wobei der Absorptionswert der Innenlage in einem Bereich von 1.000 bis 1.800%, bevorzugt 1.300 bis 1.700%, gemessen gemäß CEN/TC 172/WG 8 N 323, liegt. Dieser Bereich stellt ein Optimum zwischen dem Absorptionsvermögen des Materials einerseits und dessen Verarbeitungsfähigkeit andererseits dar. Ein besonderer Vorteil dieses Materials ist, daß es ein ausgesprochen hohes Absorptionsvermögen aufgrund der Innenlage aufweist, gleichzeitig jedoch aufgrund des für die Außenlagen verwendeten, von der Innenlage unterschiedlichem Material eine hohe Naßfestigkeit aufweist. Hierdurch wird der Widerspruch gelöst, der zwischen der Erzielung eines hohen Absorptionsvermögens eines Materials zur Herstellung von Küchenpapier oder ähnlichem und der Erzielung einer hohen Festigkeit desselben besteht. Denn je höher das Absorptionsvermögens und damit die Saugfähigkeit eines Materials ist, um so größer ist im allgemeinen dessen Volumen. Je größer jedoch das Volumen einer Einzellage eines derartigen Materials ist, um so geringer ist dessen Festigkeit, insbesondere dessen Naßfestigkeit.

Ein weiterer Vorteil des Materials ist, daß die einzelnen Lagen auf konventionellen Tissue-Maschinen hergestellt werden können, wobei lediglich die Maschinenparameter variiert werden. Hierdurch werden einerseits die Anschaffungskosten minimiert, da keine zusätzliche Durchströmtrockungsmaschine notwendig ist, andererseits verringern sich die Betriebskosten durch den geringeren Energieverbrauch einer konventionellen Tissue-Maschine im Vergleich zu einer Durchströmtrocknungs-Tissue-Maschine.

Das spezifische Volumen der Innenlage des Materials weist einen Wert von mindestens 10 cm³/g auf. Insbesondere weist die Innenlage ein spezifisches Volumen in einem Bereich von 10 bis 30 cm³/g, bevorzugt 12 bis 26 cm³/g, auf. Derartige spezifische Volumina liegen deutlich über den mit üblichen Tissue-Materialien erreichbaren und werden nur von tissueähnlichen Materialien erreicht, welche mittels Durchströmtrockgung hergestellt werden.

Vorteilhafterweise weist die Innenlage eine Bruchdehnung in einem Bereich von etwa 40 bis 80% und eine Naßdehnung in einem Bereich von 30 bis 80%, bevorzugt 40 bis 70%, gemessen jeweils gemäß DIN 54540, auf. Diese Werte sind drei- bis vierfach höher als diejenigen der Außenlagen.

Die Innenlage des Materials weist ein Flächengewicht in einem Bereich von 15 bis 35 g/cm², bevorzugt 17 bis 30 g/cm², gemessen gemäß DIN 54540, auf. Das Flächengewicht der Außenlagen liegt in einer ähnlichen Größenordnung.

Die einzelnen Lagen des erfindungsgemäßen Materials umfassen Frischzellstoff und/oder Recyclingmaterial oder eine Mischung daraus, insbesondere besteht die Innenlage aus Frischzellstoff oder Recyclingmaterial oder einer Mischung daraus.

Insbesondere ist die Innenlage des Materials aus tissueähnlichem Material, bevorzugt Zellstoffwatte. Zellstoffwatte weist eine außerordentlich hohe Saugfähigkeit auf. Zudem kann Zellstoffwatte auf herkömmlichen Tissue-Maschinen bei Vornahme von Modifikationen an diesen hergestellt werden. Die Außenlagen des erfindungsgemäßen Materials sind vorteilhafterweise aus herkömmlichem Tissue-Material. Dieses herkömmliche Tissue-Material weist eine hohe Naßfestigkeit auf und ermöglicht so in Kombination mit der Innenlage den Widerspruch zwischen hoher Naßfestigkeit und hohem Absorptionsvermögen aufzulösen.

Erfindungsgemäß weist die Innenlage ein um mindestens 100% höheren Absorptionswert, gemessen gemäß CEN/TC 172/WG 8 N 323, auf als die Außenlagen. Maximal ist der Absorptionswert der Innenlage um etwa 300% höher als derjenige der Außenlagen. Optimalerweise liegt die Differenz des Absorptionswertes zwischen der Innenlage und den Außenlagen in einem Bereich von etwa 140 bis 260%, bevorzugt 160 bis 240%. Diese Unterschiede der Außen- und Innenlagen in ihrem Absorptionswert führen vorteilhafterweise zu einer verbesserten Griffigkeit des erfindungsgemäßen Materials. Denn der Großteil der von dem Material aufgesogenen Flüssigkeit wird von der Innenlage aufgesogen, wohingegen die Außenlagen nur eine geringere Menge an Flüssigkeit aufzunehmen in der Lage sind. Durch den geringeren Feuchtigkeitsgehalt der Außenlagen bei Inkontaktbringen mit Flüssigkeit des erfindungsgemäßen Materials ist dieses für den Anwender im nassen Zustand angenehmer zu benutzen und zu greifen. Insbesondere wird hierdurch vermieden, daß beim Aufwischen von Flüssigkeit mittels des erfindungsgemäßen Materials die Bildung von Restflüssigkeitswischspuren vermieden wird, welche insbesondere bei einem Wischen mit erhöhtem Druck auftreten.

Die Erfindung betrifft weiterhin ein Verfahren zur Herstellung des erfindungsgemäßen Materials, bei welchem die einzelnen Lagen desselben von je einer Abrollung abgerollt werden und anschließend in einem Prägeschritt mittels eines Prägewerkes verbunden werden. Insbesondere kann hierbei vorgesehen werden, daß eine Außenlage mit wenigstens einer Innenlage vordoubliert wird. Durch die Vordoublierung wird vorteilhafterweise erreicht, daß eine optimale Verbindung zwischen den Außenlagen und der Innenlage bei der Herstellung des Materials erreicht wird. Zudem wird erreicht, daß der Zug der Tissue-Maschine auf die Außenlage verlagert wird, und daher Produktionsstopps durch Reißen der Materialbahnen reduziert werden. Zusätzlich zur Vordoublierung kann zwischen der Außenlage und Innenlage auch Leim aufgebracht werden, wodurch eine weitere Verbesserung der Verarbeitung des Materials erreicht wird. Die Außenlage dient hierbei als Träger für die Innenlage.

Vorteilhafterweise wird das Verfahren derart durchgeführt, daß in einem ersten Prägeschritt eine erste Außenlage mit mindestens einer Innenlage verbunden wird und anschließend in einem zweiten Prägeschritt dieser Verbund der der ersten Auβenlage entgegengesetzten Seite mit einer zweiten Außenlage verbunden wird.

Vor dem Prägeschritt werden bevorzugt die einzelnen Lagen durch eine Beleimungsvorrichtung mit einem Leim versehen. Hierbei ist vorteilhafterweise vorgesehen, daß durch die Beleimungsvorrichtung der Leim kontaktlos auf die Lagen aufgetragen wird. Durch die kontaktlose Auftragung des Leimes auf die einzelnen Lagen wird vorteilhafterweise vermieden, daß die empfindlichere Innenlage bei der Beleimung beschädigt wird, beispielsweise durch die bei einer Kontaktbeleimung verwendete Leimpresse.

Bevorzugt wird der Leim für die Verbindung der einzelnen Lagen ausgewählt aus einer Gruppe umfassend Carboxymethylcellulose, Polyvenylalkohole (PVA) und/oder Hotmelt-Leim. Hotmelt-Leime (Heißschmelzkleber) sind im allgemeinen wasserunlöslich und werden in speziellen Heizgeräten bei ca. 100 bis 160° C in flüssige Phase gebracht und anschließend versprüht. Es können aber auch wasserdispergierbare Heißschmelzkleber vorteilhaft verwendet werden, da diese eine Verbesserung der Haftung ergeben sowie Verletzungen des Materials bei Kontaktieren mit heißem, wasserlöslichem Schmelzkleber vermieden werden.

Die Oberfläche der Innenlage des erfindungsgemäßen Materials wird vorteilhafterweise auf einer konventionellen Tissue-Maschine mittels eines Kreppschabers vergrößert. Nach dem Verfahren kann vorgesehen sein, daß die mittels einer herkömmlichen Tissue-Maschine hergestellten Außen- und Innenlagen vor oder nach der Aufrollung derselben sofort anschliessend in eine Konvertier-Maschine eingeführt werden, mittels welcher die Endprodukte hergestellt werden. Bevorzugt wird als Kreppschaber ein Stahlschaber oder ein keramikbeschichteter Stahlschaber verwendet. Der Kreppschaber ist vorteilhafterweise auf einen Winkel in einem Bereich von 80° bis von 90° geschliffen, wobei weiterhin vorgesehen sein kann, daß die auf den Trockenzylinder der Tissue-Maschine hin ausgerichtete Anlagekante des Kreppschabers gebrochen wird. Hierdurch wird vorteilhafterweise erreicht, daß die Innenlage eine Hochkreppung erhält und dadurch hochsaugfähig ist. Die solchermaßen hergestellte Innenlage weist ein ausgesprochen hohes Absorptionsvermögen für Wasser auf.

Von besonderem Vorteil ist es, wenn als Anstellwinkel des Kreppschabers zum Zylinder der Tissue-Maschine ein Wert in einem Bereich von 15 bis 25°, bevorzugt 17 bis 20°, gewählt wird. Die besten Kreppergebnisse werden hierbei bei etwa 18° erzielt.

Erfindungsgemäß wird die Innenlage bei einer Differenzgeschwindigkeit der Tissue-Maschine zwischen Papiersieb und Trägerfilz derselben in einem Bereich von 5 bis 10% hergestellt. Hierdurch wird eine weitere Steigerung des Absorptionsvermögens der Innenlage erreicht. Die Verbindung zwischen der speziellen Kreppschabereinstellung und der vorstehend beschriebenen Differenzgeschwindigkeit ermöglicht es somit, Materialien herzustellen, welche ein ausgesprochen hohes Absoprtionsvermögen aufweisen und dabei auf konventionellen Tissue-Maschinen herstellbar sind.

Schließlich betrifft die Erfindung die Verwendung des erfindungsgemäßen Materials als Küchenpapier, Toilettenpapier, Taschentücher, Saugeinlage, Papierhandtuch, Wischtuch und/oder Putztuch.

Diese und andere Vorteile der vorliegenden Erfindung werden im folgenden anhand der Ausführungsbeispiele und Abbildungen erläutert. Es zeigen:
- Fig. 1: eine schematische Zeichnung einer Konvertier-Maschine mit einer Einzelabrollung aller einzelnen Lagen zur Herstellung eines erfindungsgemäßen Materials;
- Fig. 2: eine Konvertier-Maschine mit einer Abrollung einer mit einer Außenlage vordoublierten Innenlage sowie einer Einzelabrollung einer Außenlage zur Herstellung des erfindungsgemäßen Materials;
- Fig. 3: eine weitere Ausführungsform einer Konvertier-Maschine zur Herstellung des erfindungsgemäßen Materials;
- Fig. 4: eine Fotografie eines herkömmlichen Tissue-Materials; und
- Fig. 5: eine Fotografie eines hochgekreppten Tissue-Materials zur Herstellung des erfindungsgemäßen Materials.

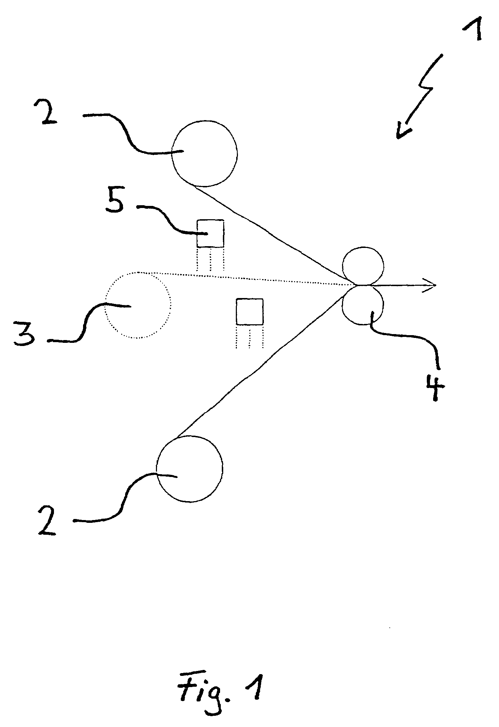

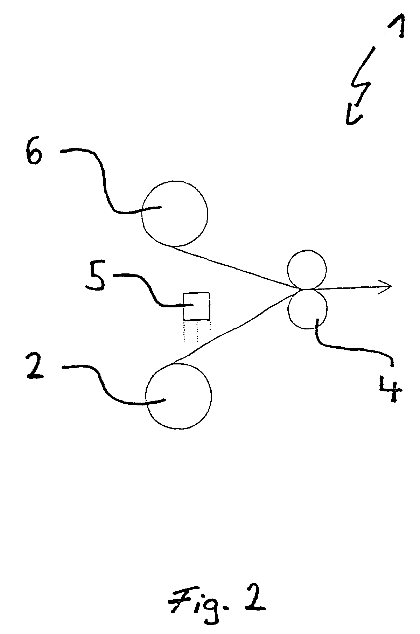

Fig. 1 zeigt schematisch den Aufbau einer Konvertier-Maschine 1 zur Herstellung des erfindungsgemäßen Materials. Die Konvertier-Maschine 1 weist insgesamt drei Einzelabrollungen auf, wobei von Einzelabrollungen 2 Material für die Außenlage und von einer Einzelabrollung 3 Material für die Innenlage abgerollt wird. Bevor die einzelnen Materialbahnen ein Prägewerk 4 durchlaufen, werden diese mittels Beleimungsvorrichtungen 5 kontaktlos mit Leim versehen. Der Leim aus den Beleimungsvorrichtungen 5 dient dazu, die Einzellagen fest miteinander zu verbinden. Mittels des Prägewerkes 4 können unterschiedliche Prägemuster auf das Material aufgebracht werden. Die Prägung bewirkt weiterhin eine Vergrößerung des spezifischen Volumens des erfindungsgemäßen Materials und gleichzeitig eine weitere Verfestigung der drei Einzellagen untereinander, wodurch die Festigkeit, insbesondere die Naßfestigkeit, des solchermaßen produzierten Materials erhöht wird.

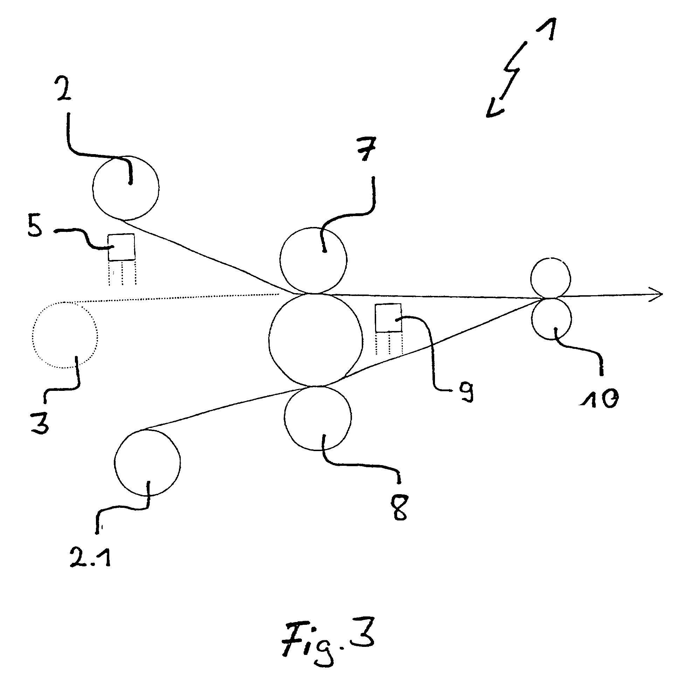

Fig. 2 zeigt eine Variante einer zur Herstellung des erfindungsgemäßen Materials verwendeten Konvertier-Maschine. Hierbei wird im Unterschied zu der in der Fig. 1 gezeigten Konvertier-Maschine von einer Einzelabrollung 6 vordoubliertes Material, bestehend aus einer Außen- und einer Innenlage, abgerollt. Dieses wird dann anschließend über das Prägewerk 4 auf der der ersten Außenlage entgegengesetzten Seite mit einer zweiten Außenlage von der Einzelabrollung 2 versehen, wobei diese vorher auf ihrer der Innenlage des vordoublierten Materials zugewandten Seite mittels der Beleimungsvorrichtung 5 kontaktlos mit Leim versehen wurde.

Nach der in der Fig. 3 gezeigten Konvertier-Maschine wird in einem ersten Schritt von der Einzelabrollung 2 eine Außenlage abgerollt und mittels eines Prägewerkes 7 mit der von der Einzelabrollung 3 abgerollten Innenlage geprägt. Vor dem Prägeschritt wird mittels der Beleimungsvorrichtung 5 kontaktlos Leim auf die der Innenseite der Außenlage zugewandten Seite der Innenlage aufgebracht. Die von der Einzelabrollung 2.1 abgerollte Außenlage wird mittels eines Prägewerkes 8 vorgeprägt und anschließend auf der der Innenlage des in dem ersten Prägeschritt geprägten Materials, bestehend aus einer Außenlage und einer Innenlage, mittels einer Beleimungsvorrichtung 9 kontaktlos mit Leim versehen. Anschließend wird die von der Einzelabrollung 2.1 abgerollte Außenlage zu einem Materialverbund mittels eines Prägewerkes 10 geprägt.

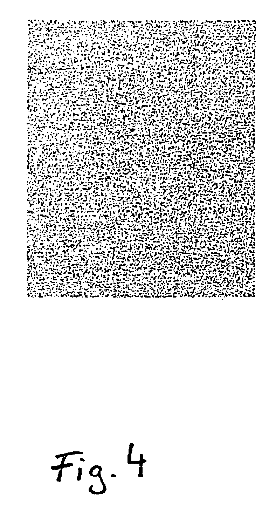

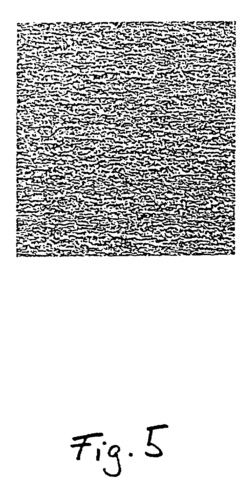

Daß sich die Außenlagen und die Innenlagen des erfindungsgemäßen Materials deutlich anhand ihrer unterschiedlichen Oberflächen unterscheiden lassen, ist aus den Fig. 4 und 5 ersichtlich. Fig. 4 zeigt ein herkömmliches Tissue-Material, welches als Außenlage gemäß der Erfindung verwendet wird. Deutlich ist eine relativ flache und dichte Struktur der Zellstoffasern dieses Materials zu erkennen. Im Unterschied hierzu zeigt Fig. 5 ein hochgekrepptes Tissue-Material, welches als Innenlage in dem erfindungsgemäßen Material Verwendung findet. Dieses weist aufgrund der Hochkreppung eine wesentlich gröbere Oberflächenstruktur auf als herkömmliches Tissue-Material.

Eine auf einer herkömmlichen Tissue-Maschine mit einem auf einem Winkel von 90° geschliffenen Stahlschaber hergestellte Innenlage, wobei der Anstellwinkel des Stahlschabers zum Zylinder 18° betrug, die Anlagekante des Stahlschabers zum Trockenzylinder der Tissue-Maschine leicht gebrochen wurde (Anpreßdruck am Zylinder der Tissue-Maschine: 2,5 bar), und wobei die Differenzgeschwindigkeit (Voreilung) zwischen dem Papiersieb und dem Trägerfilz der Tissue-Maschine 7,5% betrug, weist die folgenden in Tabelle 1 angegebenen Eigenschaften auf, wobei zur Verdeutlichung die entsprechenden Werte für die Außenlagen angegeben sind, welche aus marktüblichem, herkömmlichem Tissue-Material bestehen:

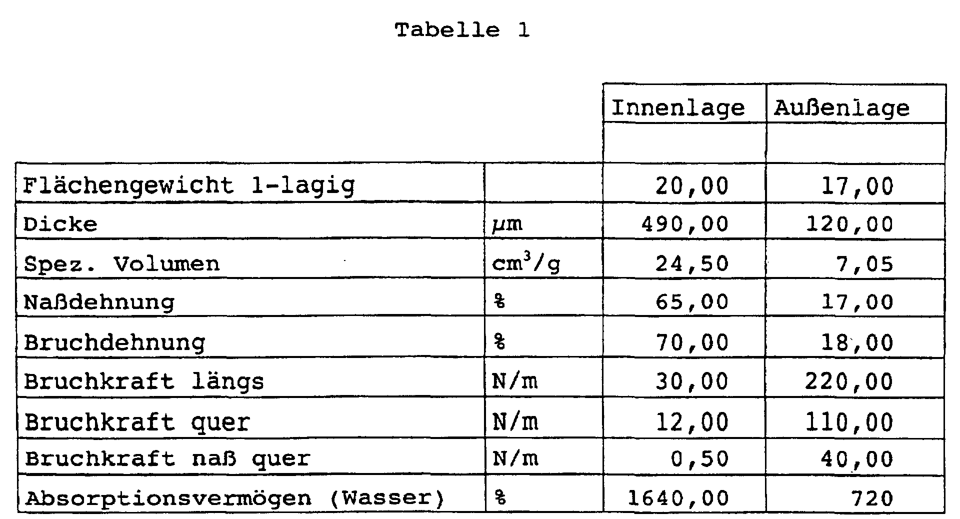

Tabelle 1: Eigenschaften einer auf einer einzelnen herkömmlichen Tissue-Maschine hergestellten Innenlage (Schabereinstellungen und Differenzgeschwindigkeiten wie im Text gegeben) und eines marktüblichen Tissue-Materials als Außenlage.

Für die Bestimmungen der in der Tabelle 1 gegebenen Parameter wurden die Proben gemäß DIN 54540 vorbereitet, die Parameter Dicke, Naßdehnung, Flächengewicht und Bruchdehnung wurden ebenfalls gemäß den Vorschriften der DIN 54540 gemessen. Das Absorptionsvermögen der Lagen von Wasser wurde gemäß CEN/TC 172/WG 8N 323 bestimmt. Das spezifische Volumen wurde aus dem Quotienten der Dicke und des Flächengewichts bestimmt.

Aus den in der Tabelle 1 angegeben Werten wird deutlich, daß die Innenlage ein mehr als doppelt so hohes Absorptionsvermögen in Wasser aufweist als die Außenlage.

Die solchermaßen hergestellte Innenlage weist eine gegenüber herkömmlichen Tissue-Materialien verminderte Bruchkraft auf. Um eine zuverlässige Verarbeitung der Innenlage zu ermöglichen und die erfindungsgemäßen Materialien herzustellen, wird daher eine Vordoublierung der Außenlage mit der Innenlage vorgenommen.

Aus zwei Außenlagen und einer Innenlage mit den in der Tabelle 1 genannten Eigenschaften wurde das erfindungsgemäße Material mittels des in Fig. 2 gezeigten Verfahrens hergestellt. Es wurde hierbei eine einzelne Innenlage verwendet. Jedoch ist es auch möglich, zwei oder mehr Innenlagen zwischen zwei Außenlagen anzuordnen. Hierbei können die Innenlagen auch mit einer Außenlage vordoubliert sein. Das solchermaßen hergestellte erfindungsgemäße Material in Form einer Küchenpapierrolle weist die in der Tabelle 2 angegebenen Eigenschaften auf, wobei das erfindungsgemäße Material verglichen wurde mit einem ebenfalls dreilagigen Material, welches aus drei Lagen eines konventionellen Tissue-Materials hergestellt wurde.

**Tabelle 2**

| | Einheit | Erfindungsgemäßes Material | Vergleichsmuster | Methode |
|---|---|---|---|---|
| Zahl der Einzellagen | | 3 | 3 | |
| Flächengewicht 3-lagig | g/m² | 52,8 | 54 | DIN 54 540 |
| Dicke | µm | 718 | 710 | DIN 54 540 |
| Spez. Volumen | cm³/g | 13,59 | 13,15 | DIN 54 540 |
| Bruchkraft 3-lagig Maschinenrichtung | N/m | 195 | 250 | DIN 54 540 |
| Bruchkraft 3-lagig Querrichtung | N/m | 91 | 125 | DIN 54 540 |
| Bruchdehnung Maschinenrichtung | % | 9,7 | 8,1 | DIN 54 540 |
| Absorptionsvermögen | % | 1580 | 800 | CEN |

Tabelle 2: Eigenschaften des erfindungsgemäßen Materials, hergestellt aus einer Innenlage und zwei Außenlagen mit den Eigenschaften gemäß Tab. 1, im Vergleich zu einer Küchenpapierrolle, hergestellt aus drei Lagen herkömmlichen Tissue-Materials (MD: längs; CD: quer).

Aus dem erfindungsgemäßen Material hergestellte Küchenpapierrollen weisen im Vergleich zu dreilagigen Küchenpapierrollen, hergestellt aus konventionellen Tissue-Materialien gemäß den in der Tabelle 2 angegebenen Eigenschaften einen um fast 100% erhöhten Wert für das Absorptionsvermögen von Wasser auf. Gleichzeitig sind die die Festigkeit des Materials beschreibenden Parameter, wie Bruchdehnung, Naßdehnung und Bruchstärke, des aus dem erfindungsgemäßen Material hergestellten Küchenpapiers in etwa demjenigen des dreilagigen, aus konventionellen Tissue-Materialien hergestellten Küchenpapiers, vergleichbar.

## Patentansprüche

1. Tissue- und/oder tissueähnliches Material zur Herstellung von Küchenpapier, Toilettenpapier, Taschentüchern, Saugeinlagen und ähnlichem, welches mindestens dreilagig ist, umfassend zwei Außenlagen und mindestens eine zwischen diesen beiden Außenlagen angeordnete Innenlage mit einem höheren Absorptionswert für Wasser als die Außenlagen, wobei der Absorptionswert der Innenlage in einem Bereich von 1000 bis 1800%, bevorzugt 1300 bis 1700%, gemessen gemäß CEN/TC 172 WG 8 N 323, liegt.

2. Material nach Anspruch 1, **dadurch gekennzeichnet, daß** die Innenlage ein spezifisches Volumen von mindestens 10cm³/g aufweist.

3. Material nach einem der vorhergehenden Ansprüche, **dadurch gekennzeichnet, daß** die Innenlage ein spezifisches Volumen in einem Bereich von 10 bis 30 cm³/g, bevorzugt 12 bis 26 cm³/g, aufweist.

4. Material nach einem der vorhergehenden Ansprüche, **dadurch gekennzeichnet, daß** die Innenlage eine Bruchdehnung in einem Bereich von etwa 40 bis 80% aufweist, gemessen gemäß DIN 54540.

5. Material nach einem der vorhergehenden Ansprüche, **dadurch gekennzeichnet, daß** die Innenlage eine Naßdehnung in einem Bereich von 30 bis 80%, bevorzugt 40 bis 70%, aufweist, gemessen gemäß DIN 54540.

6. Material nach einem der vorhergehenden Ansprüche, **dadurch gekennzeichnet, daß** die Innenlage ein Flächengewicht in einem Bereich von 15 bis 35 g/cm², bevorzugt 17 bis 30 g/cm², aufweist, gemessen gemäß DIN 54540.

7. Material nach einem der vorhergehenden Ansprüche, **dadurch gekennzeichnet, daß** die Lagen Frischzellstoff und/oder Recyclingmaterial oder eine Mischung daraus umfassen.

8. Material nach einem der vorhergehenden Ansprüche, **dadurch gekennzeichnet, daß** die Innenlage aus Frischzellstoff oder Recyclingmaterial oder einer Mischung daraus besteht.

9. Material nach einem der vorhergehenden Ansprüche, **dadurch gekennzeichnet, daß** die Innenlage aus tissueähnlichem Material, bevorzugt Zellstoffwatte, hergestellt ist.

10. Material aus einem der vorhergehenden Ansprüche, **dadurch gekennzeichnet, daß** die Außenlagen aus Tissue-Material sind.

11. Material nach einem der vorhergehenden Ansprüche, **dadurch gekennzeichnet, daß** die Innenlage einen um mindestens 100% höheren Absorptionswert, gemessen gemäß CEN/TC 172/WG 8 N 323, aufweist als die Außenlagen.

12. Verfahren zur Herstellung eines Materials gemäß einem der Ansprüche 1 bis 11, **dadurch gekennzeichnet, daß** die Lagen von je einer Abrollung (2, 3, 6) abgerollt und anschließend in einem Prägeschritt mittels eines Prägewerkes (4, 7, 8, 10) verbunden werden.

13. verfahren nach Anspruch 12, **dadurch gekennzeichnet, daß** eine Außenlage mit wenigstens einer Innenlage vordoubliert wird.

14. Verfahren nach einem der Ansprüche 12 oder 13, **dadurch gekennzeichnet, daß** in einem ersten Prägeschritt eine erste Außenlage mit mindestens einer Innenlage verbunden wird und anschließend in einem zweiten Prägeschritt dieser Verbund auf der der ersten Außenlage entgegengesetzten Seite mit einer zweiten Außenlage verbunden wird.

15. Verfahren nach einem der vorhergehenden Ansprüche, **dadurch gekennzeichnet, daß** vor dem Prägeschritt die Lagen durch eine Beleimungsvorrichtung (5, 9) mit einem Leim versehen werden.

16. Verfahren nach einem der vorhergehenden Ansprüche, **dadurch gekennzeichnet, daß** durch die Beleimungsvorrichtung (5, 9) der Leim kontaktlos auf die Lagen aufgetragen wird.

17. Verfahren nach einem der vorhergehenden Ansprüche, **dadurch gekennzeichnet, daß** der Leim ausgewählt wird aus einer Gruppe umfassend Carboxymethylcellulose, PVA und/oder wasserdispergierbaren und/oder wasserunlöslichen Hotmelt-Leim.

18. Verfahren nach einem der vorhergehenden Ansprüche, **dadurch gekennzeichnet, daß** die Oberfläche der Innenlage auf einer Tissue-Maschine mittels eines Kreppschabers vergrößert wird.

19. Verfahren nach einem der vorhergehenden Ansprüche, **dadurch gekennzeichnet, daß** als Kreppschaber ein Stahlschaber oder ein keramikbeschichteter Stahlschaber verwendet wird.

20. Verfahren nach einem der vorhergehenden Ansprüche, **dadurch gekennzeichnet, daß** der Kreppschaber auf einen Winkel in einem Bereich von 80° bis 90° geschliffen wird.

21. Verfahren nach einem der vorhergehenden Ansprüche, **dadurch gekennzeichnet, daß** die auf den Trockenzylinder der Tissue-Maschine hin ausgerichtete Anlagekante des Kreppschabers gebrochen wird.

22. Verfahren nach einem der vorhergehenden Ansprüche, **dadurch gekennzeichnet, daß** als Anstellwinkel des Kreppschabers am Zylinder ein Wert in einem Bereich von 15 bis 25°, bevorzugt 17 bis 20°, gewählt wird.

23. Verfahren nach einem der vorhergehenden Ansprüche, **dadurch gekennzeichnet, daß** die Innenlage bei einer Differenzgeschwindigkeit der Tissue-Maschine zwischen Papiersieb und Trägerfilz in einem Bereich von 5 bis 10% hergestellt wird.

24. Verwendung eines Materials nach einem der Ansprüche 1 bis 11 als Küchenpapier, Toilettenpapier, Taschentücher, Saugeinlage, Papierhandtuch, Wischtuch und/oder Putztuch.

## Claims

1. Tissue and/or tissue-like material for the production of kitchen paper, toilet paper, handkerchiefs, absorbent inlays and the like, consisting of at least three plies, which comprise two outer plies and at least one inner ply, which is arranged between these two outer plies and has a higher absorption factor for water than the outer plies, whereby the absorption factor of the inner ply is in a range of 1000 to 1800 %, preferred 1300 to 1700 %, measured according to CEN/TC 172 WG 8 N 323.

2. Material according to claim 1, **characterised in that** the inner ply has a specific volume of at least 10 cm³/g.

3. Material according to one of the afore mentioned claims, **characterised in that** the inner ply has a specific volume in a range of 10 to 30 cm³/g, preferred 12 to 26 cm³/g.

4. Material according to one of the afore mentioned claims, **characterised in that** the inner ply has a stretch at break in a range of about 40 to 80 %, measured according to DIN 54540.

5. Material according to one of the afore mentioned claims, **characterised in that** the inner ply has a wet expansion in a range of 30 to 80 %, preferred 40 to 70 %, measured according to DIN 54540.

6. Material according to one of the afore mentioned claims, **characterised in that** the inner ply has a basis weight in a range of 15 to 35 g/cm², preferred 17 to 30 g/cm², measured according to DIN 54540.

7. Material according to one of the afore mentioned claims, **characterised in that** the plies comprise fresh chemical pulp and/or recycling material or a mixture thereof.

8. Material according to one of the afore mentioned claims, **characterised in that** the inner ply consists of fresh chemical pulp or recycling material or a mixture thereof.

9. Material according to one of the afore mentioned claims, **characterised in that** the inner ply is made of a tissue-like material, preferred cellulose wadding.

10. Material according to one of the afore mentioned claims, **characterised in that** the outer plies are made of a tissue material.

11. Material according to one of the afore mentioned claims, **characterised in that** the absorption factor of the inner ply is by at least 100 % higher than the absorption factor of the outer plies, measured according to CEN/TC 172/WG 8 N 323.

12. Method to produce a material according to one of the claims 1 to 11, **characterised in that** the plies are unrolled each from a corresponding unwind (2, 3, 6) and subsequently bonded in one embossing step by means of an embossing unit (4, 7, 8, 10).

13. Method according to claim 12, **characterised in that** an outer ply is pre-doubled with at least one inner ply.

14. Method according to one of the claims 12 or 13, **characterised in that** in a first embossing step a first outer ply is bonded with at least one inner ply and subsequently in a second embossing step this bond is bonded with a second outer ply on the side opposed to the first outer ply.

15. Method according to one of the afore mentioned claims, **characterised in that** before the embossing step, the plies are coated with glue by means of a glue spreading device (5, 9).

16. Method according to one of the afore mentioned claims, **characterised in that** by means of the glue spreading device (5, 9) the glue is applied onto the plies without contacting the same.

17. Method according to one of the afore mentioned claims, **characterised in that** the glue is selected from a group comprising carboxymethylcellulose, PVA and/or water-dispersible and/or water-insoluble hot-melt glue.

18. Method according to one of the afore mentioned claims, **characterised in that** the surface of the inner ply is increased on a tissue machine by means of a crepe scraper.

19. Method according to one of the afore mentioned claims, **characterised in that** a steel scraper or a steel scraper coated with ceramics is used as crepe scraper.

20. Method according to one of the afore mentioned claims, **characterised in that** the crepe scraper is cut to an angle in a range of 80° to 90°.

21. Method according to one of the afore mentioned claims, **characterised in that** the lay-on edge of the crepe scraper, which is directed towards the drying cylinder of the tissue machine, is broken.

22. Method according to one of the afore mentioned claims, **characterised in that** a factor in a range of 15 to 25°, preferred 17 to 20°, is selected as a setting angle of the crepe scraper.

23. Method according to one of the afore mentioned claims, **characterised in that** the inner ply is produced at a difference velocity of the tissue machine between paper sieve and carrier felt, which is in a range of 5 to 10 %.

24. Use of the material according to one of the claims 1 to 11 as kitchen paper, toilet paper, handkerchiefs, absorbent inlays, paper towel, wipe and/or floor cloth.

## Revendications

1. Papier ménager et/ou matière de type papier ménager destiné à la fabrication de papier essuie-tout, de papier hygiénique, de mouchoirs en papier, de garnitures hygiéniques et similaires, qui est au moins triple couche, comprenant deux couches extérieures et au moins une couche intérieure avec une valeur d'absorption de l'eau plus élevée que les couches extérieures disposée entre ces deux couches extérieures, la valeur d'absorption de la couche intérieure, mesurée selon CEN/TC 172 WG 8 N 323, étant située dans une plage de 1000 à 1800 %, de préférence de 1300 à 1700 %.

2. Matière selon la revendication 1, **caractérisée en ce que** la couche intérieure présente un volume spécifique d'au moins 10 cm³/g.

3. Matière selon l'une quelconque des revendications précédentes, **caractérisée en ce que** la couche intérieure présente un volume spécifique situé dans une plage de 10 à 30 cm³/g, de préférence de 12 à 16 cm³/g.

4. Matière selon l'une quelconque des revendications précédentes, **caractérisée en ce que** la couche intérieure présente un allongement à la rupture situé dans une plage d'environ 40 à 80%, mesuré selon DIN 54540.

5. Matière selon l'une quelconque des revendications précédentes, **caractérisée en ce que** la couche intérieure présente un allongement à l'état humide situé dans une plage de 30 à 80%, de préférence de 40 à 70%, mesuré selon DIN 54540.

6. Matière selon l'une quelconque des revendications précédentes, **caractérisée en ce que** la couche intérieure présente un grammage situé dans une plage de 15 à 35 g/cm², de préférence de 17 à 30 g/cm², mesuré selon DIN 54540.

7. Matière selon l'une quelconque des revendications précédentes, **caractérisée en ce que** les couches contiennent de la cellulose fraîche et/ou de la matière de recyclage ou un mélange de celles-ci.

8. Matière selon l'une quelconque des revendications précédentes, **caractérisée en ce que** la couche intérieure est constituée de cellulose fraîche ou de matière de recyclage ou d'un mélange de celles-ci.

9. Matière selon l'une quelconque des revendications précédentes, **caractérisée en ce que** la couche intérieure est fabriquée à partir d'une matière de type papier ménager, de préférence à partir d'ouate de cellulose.

10. Matière selon l'une quelconque des revendications précédentes, **caractérisée en ce que** les couches extérieures sont constituées de papier ménager.

11. Matière selon l'une quelconque des revendications précédentes, **caractérisée en ce que** la couche intérieure présente une valeur d'absorption, mesurée selon CEN/TC 172/WG 8 N 323, supérieure d'au moins 100% aux couches extérieures.

12. Procédé de fabrication d'une matière selon l'une quelconque des revendications 1 à 11, **caractérisé en ce que** les couches sont déroulées à chaque fois d'un dérouloir (2, 3, 6), puis assemblées pendant une étape de gaufrage au moyen d'un mécanisme de gaufrage (4, 7, 8, 10).

13. Procédé selon la revendication 12, **caractérisé en ce qu'**une couche extérieure est prédoublée avec au moins une couche intérieure.

14. Procédé selon l'une quelconque des revendications 12 ou 13, **caractérisé en ce qu'**une première couche extérieure est assemblée avec au moins une couche intérieure pendant une première étape de gaufrage, puis cet ensemble est assemblé sur la face opposée à la première couche extérieure avec une deuxième couche extérieure pendant une deuxième étape de gaufrage.

15. Procédé selon l'une quelconque des revendications précédentes, **caractérisé en ce qu'**avant l'étape de gaufrage, les couches sont encollées à l'aide d'un dispositif d'encollage (5, 9).

16. Procédé selon l'une quelconque des revendications précédentes, **caractérisé en ce qu'**avec le dispositif d'encollage (5, 9), la colle est déposée sans contact sur les couches.

17. Procédé selon l'une quelconque des revendications précédentes, **caractérisé en ce que** la colle est choisie dans un groupe comprenant la carboxyméthylcellulose, le PVA et/ou des colles thermofusibles dispersables dans l'eau et/ou insolubles dans l'eau.

18. Procédé selon l'une quelconque des revendications précédentes, **caractérisé en ce que** la surface de la couche intérieure est agrandie sur une machine à papier ménager au moyen d'une lame de crêpage.

19. Procédé selon l'une quelconque des revendications précédentes, **caractérisé en ce qu'**en guise de lame de crêpage est utilisée une lame en acier ou une lame en acier revêtue de céramique.

20. Procédé selon l'une quelconque des revendications précédentes, **caractérisé en ce que** la lame de crêpage est aiguisée selon un angle situé dans une plage de 80 à 90°.

21. Procédé selon l'une quelconque des revendications précédentes, **caractérisé en ce que** l'arête d'appui de la lame de crêpage orientée vers le cylindre sécheur de la machine à papier ménager est abattue.

22. Procédé selon l'une quelconque des revendications précédentes, **caractérisé en ce qu'**en guise d'angle d'inclinaison de la lame de crêpage sur le cylindre est choisie une valeur située dans une plage de 15 à 25°, de préférence de 17 à 20°.

23. Procédé selon l'une quelconque des revendications précédentes, **caractérisé en ce que** la couche intérieure est fabriquée avec une vitesse différentielle de la machine à papier ménager entre le moule pour papier et le feutre de support située dans une plage de 5 à 10%.

24. Utilisation d'une matière selon l'une quelconque des revendications 1 à 11 comme papier essuie-tout, papier hygiénique, mouchoir en papier, garniture hygiénique, essuie-mains en papier, torchon et/ou chiffon.
